# EUROPEAN PATENT APPLICATION

(11) **EP 1 516 924 A1**
(43) Date of publication of application: **23.03.2005**
(21) Application number: 03380203.4
(22) Date of filing: 17.09.2003
(51) Int. Cl.: C12N 5/06, A01K 67/027

(54) **GENERATION OF HUMAN EMBRYONIC STEM CELLS FROM TRIPLOID ZYGOTES**

(71) Applicant: Fundacion IVI para el Estudio de la reproduccion Humana (FIVIER), 46020 Valencia (ES); CNIC, Fundacion Centro Nacional de Investigaciones Cardiovasculares Carlos III, 28029 Madrid (ES)
(72) Inventor: Simon Valles, Carlos, 46020 Valencia (ES); Pellicer Martinez, Antonio, 46020 Valencia (ES); Esplugues Mota, Juan, Vicente, 46020 Valencia (ES)
(74) Representative: Primo de Rivera y Urquijo, Jose Antonio

(57) **Abstract**

Triploid zygotes arise in ∼5% of *in vitro* fertilisations, but the excess set of haploid chromosomes means that they cannot develop into human beings, and so such zygotes are not used during fertility treatment. The invention provides a method for preparing embryonic stem cells, comprising the steps of: (a) providing a tripronucleated zygote; (b) removing one of the pronuclei to provide a diploid heteroparental zygote; (c) culturing the diploid zygote to produce a blastocyst; (d) obtaining one or more cells from the inner cell mass of the blastocyst; and (e) culturing the cells obtained from the inner cell mass to obtain embryonic stem cells. The stem cells may be differentiated into cell types of interest.

## Description

All documents cited herein are incorporated by reference in their entirety.

### TECHNICAL FIELD

This invention is in the stem cell field. In particular, it relates to the derivation of embryonic stem cells from dispermic triploid zygotes, which zygotes arise during *in vitro* fertilisation but are unsuitable for implantation.

### BACKGROUND ART

The production and potential of embryonic stem (ES) cells have been widely reported for many organisms, including humans [*e.g.* see references 1 to 12]. Despite the positive opportunities which these cells offer, however, their origin has resulted in ethical concerns being raised by many groups [*e.g.* refs. 13-15]. It is an object of the invention to provide methods for producing ES cells which overcome at least some of these ethical issues. It is a further object of the invention to provide ways of using these ES cells.

### DISCLOSURE OF THE INVENTION

The invention is based on using triploid zygotes [16,17] as a starting point for preparing ES cells. Triploid zygotes arise in ~5% of *in vitro* fertilisations (IVF), but the excess set of haploid chromosomes means that they cannot develop into human beings [18]. Although the excess nucleus can be removed to restore a diploid state, such manipulated and/or pharmacologically-treated zygotes cannot be implanted because of the risks involved (*e.g.* high levels of mosaicism [90% - ref. 19]) and their clinical use is ethically and/or legally banned by many authorities.

The IVF procedure thus gives rise to a significant proportion of zygotes which are embryologically non-viable in their un-manipulated state and ethically/legally non-viable in their manipulated state. The inventors have found a use for these zygotes in the production of ES cells. These ES cells can be distinguished from those produced using known techniques because they are produced from zygotes which would not have been implanted and thus could not have developed into fetuses or beyond.

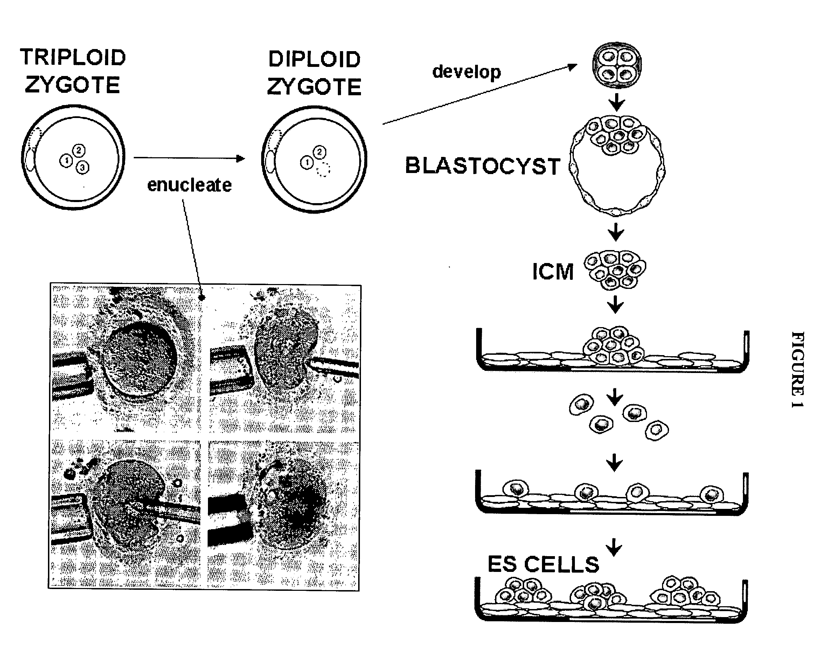

Thus the invention provides a process for preparing embryonic stem cells, comprising the steps of: (a) providing a tripronucleated zygote; (b) removing one of the pronuclei to provide a diploid heteroparental zygote; (c) culturing the diploid zygote to produce a blastocyst; (d) obtaining one or more cells from the inner cell mass of the blastocyst; and (e) culturing the cells obtained from the inner cell mass to obtain embryonic stem cells. The invention also provides ES cells obtained and/or obtainable by this process, which is illustrated in Figure 1.

### The tripronucleated zygote

The tripronucleated zygote used in step (a) is preferably one obtained from IVF. Polyspermic fertilisation occurs in ~5% of IVF zygotes, and dispermic zygotes are suitable for use with the invention. Zygotes containing three pronuclei can easily be distinguished from normal diploid zygotes e.g. by visual evaluation 16-18 hours after insemination. Pseudopronuclei can also be distinguished from true pronuclei, as they lack nucleoli.

As an alternative, polyspermic nuclei may also be obtained by intracytoplasmic sperm injection.

The zygote is preferably a human zygote, although the method of the invention can also be applied to zygotes from other organisms, particularly mammals e.g. non-human primates, cows, sheep, pigs, goats, horses, mice, rats, *etc.*

The zygote may be treated with compounds to relax the cytoskeleton. Preferred compounds induce depolymerisation of actin and tubulin filaments, and include the cytochalasins (*e.g.* type B) and colcemid (*e.g.* demecolcine). Where relaxant(s) is/are used, it is preferred to treat the zygotes prior to epronucleation, but treatment can be used before, during or after removal of the extra pronucleus. However, the use of cytoskeletal relaxants is not essential for success [17].

Cytoskeletal relaxants are generally cytotoxic, but it has been found that a brief period of exposure can be tolerated and can improve downstream survival rates. A typical concentration of cytochalasin B is between 1-20µg/ml, preferably between 5-10µg/ml, and more preferably about 7.5µg/ml (~16µM). A typical concentration of demecolcine is between 0.01-0.3M, preferably between 0.05-0.15M, and more preferably about 0.1M (~37µg/ml). Zygotes are typically exposed to the chemicals for less than about 15 minutes *e.g.* less than 10, 9, 8, 7, 6, 5, 4, 3, 2 or 1 minute. Treatment for between 1 and 5 minutes is preferred. It has been found that a treatment with a concentration *x* for a short time *y* is effective, whereas concentration *x*/*2* for time *2y* can be toxic.

### Epronucleation

In a tripronucleated zygote containing two sperm-derived pronuclei and one ovum-derived pronucleus, one of the sperm-derived pronuclei must be removed to leave a normal heteroparental diploid karyotype. It has been found that the pronucleus furthest away from the second polar body in the zygote is usually of paternal origin [16,19], and so this is preferably the pronucleus which is removed in step (b). Thus step (b) will typically involve the steps of identifying the positions of the zygote's pronuclei in relation to the second polar body, followed by removal of the distal pronucleus. Zygotes prepared in this way have been shown to progress normally through *in vitro* development up to at least the blastocyst stage.

To identify the distal pronucleus, tripronucleated zygotes can be held with a holding pipette and rotated and re-positioned in order to identify the relative positions of the three pronuclei and the two polar bodies. The pronuclei are usually arranged triangularly near the centre of the ooplasm.

The excess pronucleus is preferably removed by physical manipulation e.g. by the use of a micropipette. A micropipette can be inserted through the zona pellucida into the vitellus and positioned near the supernumerary pronucleus and, by the use of negative pressure, the pronucleus can be picked up and withdrawn from the syngamy area to the oolema. Aspiration can then be used to place the pronucleus (typically with some cytoplasm) in the micropipette for enucleation.

Guidance of pipettes can be assisted by visualising spindles using a colposcope.

After removal of pronuclei, zygotes may be washed and maintained in culture to assess survival and viability before proceeding to the blastocyst stage.

Removal of the excess pronucleus may be accompanied by removal of the associated centrosome and/or pronuclear membrane.

The invention can also use 4-ploid, 5-ploid, 6-ploid, *etc.* zygotes, with the appropriate number of excess pronuclei being removed to give a diploid cell, but such polyploidy zygotes are less common than triploid zygotes and the required manipulation is more complex.

### Production of a blastocyst

During development, a zygote divides to reach the 8-cell stage, which divides into a 32-cell stage known as a morula. After 5-6 days, the morula undergoes cavitation to form a blastocyst comprising an inner cell mass (ICM) and a layer of trophoectoderm cells which surrounds a fluid-filled cavity known as the blastocoel. The ICM is located within the blastocoel.

Heteroparental diploid zygotes can develop into blastocysts either *in vitro* or *in vivo.* Techniques for achieving this developmental progression are well known in the art *e.g.* culture on monolayers. In a preferred method, zygotes are co-cultured up to the blastocyst stage with endometrial epithelial cells [20], which give a high efficiency of blastocyst development.

To facilitative further downstream manipulation, the zona pellucida may be removed from blastocysts *e.g.* by brief incubation in Tyrode's solution, use of pronase, *etc.*

Techniques for obtaining cells from the ICM of blastocysts and for removing trophoectoderm are well known in the art, and include microsurgery or immunosurgery methods as well as laser ablation.

It is preferred to use all or substantially all of the ICM for preparing a blastocyst.

### Preparing ES cells from the inner cell mass

ES cells can be obtained by culturing ICM cells, and techniques for doing so are well known [*e.g.* see refs. 1 to 9]. Typically, ES production involves *in vitro* culture of ICM cells on a feeder layer of cells, such as inactivated mouse fibroblasts for human ES cells, with re-plating of the ICM-derived cells usually being required. To avoid possible xeno-contamination, however, it is preferred to use a feeder layer from the same organism as the ES cells, and various human feeder cells have been disclosed for maintaining human ES cells in an undifferentiated state e.g. adult marrow cells [21], fetal muscle [22], fetal skin [22], adult fallopian tube [22], foreskin [23,24], tissue-derived stromal cells [25], embryonic fibroblasts [24], placental fibroblasts, *etc.* Feeder cells will usually be inactivated, for example by myomysin treatment or, more preferably, by irradiation (*e.g.* gamma radiation). Feeder-free maintenance conditions have also been described based on artificial extracellular matrices *e.g.* using dishes coated with Matrigel™ (solubilised basement membrane preparation extracted from EHS mouse sarcoma, rich in laminin, collagen IV, heparin sulphate proteoglycans and entactin [26]), fibronectin, laminin, *etc.* [*e*.*g*. see refs. 27 to 29] in order to provide an artificial extracellular matrix, and sometimes using a conditioned medium.

The medium used to maintain ES cells will generally be buffered and/or isotonic, and it will contain appropriate nutrients for the cells *(e.g.* a source of amino acids, vitamins, *etc.).* The medium may contain serum, or it may be serum-free [24]. If serum is not used, a serum replacement may be used instead (*e.g.* containing FGF). The absence of serum avoids the introduction of undefined or random factors which may promote the formation of non-desired cell types. Conditioned media may be used (*e.g.* fibroblast-conditioned medium), preferably after having cells removed. A common medium used to maintain ES cells is KO-DMEM [30], which can be supplemented by KO-SR [31].

Human ES cells obtained by the method of the invention will typically have one or more of the following characteristics: a stable karyotype; 23 pairs of chromosomes (including XX or XY); prolonged ability to divide symmetrically without differentiation; an ability to give rise to differentiated cell types from all three primary germ layers [42] *i.e.* ectoderm, endoderm and mesoderm; prolonged telomerase activity; display of markers of non-differentiation (*e.g.* Oct-4 [69], stage-specific embryonic antigens SSEA-3 and SSEA-4, alkaline phosphatase, *etc.),* which may be detected *e.g*. by RT-PCR and/or by immunohistochemistry; non-tumorigenic; *etc.*

ES cells of the invention are preferably pluripotent. They will typically not be totipotent *e.g.* they may not be able to give rise to another embryo.

### Differentiation of ES cells

ES cells can be differentiated into other cell types for further use [e.g. see refs. 32-36]. Thus the invention provides a process for preparing a desired cell, comprising steps (a) to (e) as described above, and the further step of: (f) differentiating the ES cell into the desired cell. The invention also provides cells obtained and/or obtainable by this method. Unlike the starting ES cells, the differentiated cells cannot proliferate indefinitely *in vitro.*

Step (f) may involve the production of an embryoid body (EB). EBs are aggregates of cells which form when ES cells, embryonic germ (EG) cells, or embryonal carcinoma (EC) cells are grown in suspension culture (*e.g.* when plated on a non-adhesive surface that prevents cell attachment). They are widely recognised in the art and can be produced routinely [*e.g.* refs. 37 to 45] for organisms including humans, mice and monkeys. The production of some cell types from ES cells may need to pass through an intermediate EB stage. Thus step (f) may comprise a period of suspension culture, typically in a vessel which does not favour cell attachment (*e.g.* a petri dish). It is also possible to use techniques such as microbead culture [40]. If the starting ES cells are in adherent culture, they can be disengaged from a tissue culture surface prior to step (a) by methods involving the use of mechanical disaggregation, enzymatic treatment (*e.g.* with trypsin, papain, collagenase, *etc.),* and/or metal ion chelators *(e.g.* EDTA, EGTA), *etc.* Differentiation methods designed to avoid EB formation are described in reference 46. Methods for enhancing EB formation are described in reference 47.

Step (f) preferably produces cells from the endoderm, mesoderm or ectoderm, or germ cells *(e.g.* ova, oocytes, spermatozoa, spermatocytes, *etc.).* The desired cells include, but are not limited to: neural cells; cardiac cells; intestinal cells; stomach cells; oesophageal cells; retinal cells; corneal cells; cartilage; bone cells; hematopoietic cells; and muscle cells. Other desired cells include, but are not limited to: lung cells; kidney cells; pancreatic cells; islet cells; glucose-sensitive insulin-secreting cells; brain cells; epithelial cells; endothelial cells; epidermis; cardiomycocytes; hematopoietic progenitors; yolk sac; skeletal myocytes; smooth muscle cells; fibroblasts; adipocytes; chondrocytes; melanocytes; neural precursors; neurons, including dopaminergic neurons, serotonergic neurons, cholinergic neurons, GABAergic neurons, sensory neurons, and motor neurons; glia; astrocytes; oligodendrocytes; hepatocytes; osteocytes; osteoblasts; monocytes; dendritic cells; lymphocytes; erythrocytes; leukocytes; NK cells; macrophages; erythroid cells; granulocytes; megakaryocytes; pneumocytes; comeocytes; trophoblast; keratinocytes; testis; ovary; cell lines; conjunctiva; limbal stem cells; odontoblasts; mucosal cells; lachrymal cells; *etc.* The invention provides all such cells.

The cells produced in step (f) may express one or more of the following markers: core binding factor α1, collagen 1, osteocalcin, opteopontin, bone sialoprotein, parathyroid hormone receptor, bone/liver/kidney alkaline phosphatase, microtubule-associated protein 2, β_{III} tubulin, neurofilament glial fibrillary acidic protein, galactocerebroside, myelin basic protein, nestin, cardiac-specific troponin, sMHC, tropomyosin, α-actinin, desmin, creatine kinase-MB, myoglobin, GATA-4, MEF-2, Nkx2.5, atrial natriuretic factor, cardiac troponin T, albumin, α₁-antitrypsin, CD18' cytokeratin 18, cytokeratin 8, cytokeratin 19, vimentin, nestin, polysialylated-NCAM, A2B5, PECAM1, VE-cad, GATA-2, CD34, pdx1, Ngn3, insulin, IAPP, Nkx6.1, transcription factor AP-2, msh homeobox homolog 2, suppressor of cytokine signalling 3, GATA binding protein 2, GATA binding protein 3, hairy/enhancer-of-split related with YRPW motif 1, keratin, aggrecan, type II collagen, osteocalcin, type X collagen, etc.

Expression of these markers may be detectable by RT-PCR or, more preferably, by immunocytochemistry. When a marker is expressed then labelling with a fluorescent anti-marker antibody will generally give a signal shift of ≥ ½ log in FACS.

Preferred cells of the invention are: osteoblasts that are Oct4⁻, core binding factor α1⁺, collagen 1⁺, osteocalcin⁺, opteopontin⁺, bone sialoprotein⁺, parathyroid hormone receptor⁺ and/or bone/liver/kidney alkaline phosphatase⁺ [84]; neurons that are microtubule-associated protein 2⁺, β_{III} tubulin⁺ and/or neurofilament⁺ [62]; astrocytes that are glial fibrillary acidic protein⁺ [62]; oligodendrocytes that are galactocerebroside⁺ and/or myelin basic protein⁺ [62]; neural precursors that are nestin⁺ [62]; beating cardiomyocytes that are cardiac-specific troponin⁺, sMHC⁺, tropomyosin⁺, α-actinin⁺, desmin⁺, myogenin⁻, creatine kinase-MB⁺, myoglobin⁺, GATA-4⁺, MEF-2⁺, Nkx2.5⁺, atrial natriuretic factor⁺ and/or cardiac troponin T⁺ [73]; hepatocytes that are albumin⁺, α₁-antitrypsin⁺, CD18^{+,} cytokeratin 18⁺, cytokeratin 8⁺, cytokeratin 19⁺, vimentin⁺ and/or α-fetoprotein⁻ [54,55]; neural progenitors that are nestin⁺, polysialylated-NCAM⁺ and/or A2B5⁺ [58]; endothelial cells that are PECAM1⁺, VE-cad⁺, GATA-2⁺ and/or CD34⁺ [69,70]; insulin-secreting cells that are pdx1⁺ Ngn3⁺, insulin⁺, IAPP⁺ and/or Nkx6.1⁺ [79]; trophoblast cells that are transcription factor AP-2⁺, msh homeobox homolog 2⁺, suppressor of cytokine signalling 3⁺, GATA binding protein 2⁺, GATA binding protein 3⁺ and/or hairy/enhancer-of-split related with YRPW motif 1⁺ [80]; keratinocytes that are keratin⁺ [82]; and chondrocytes that are aggrecan⁺, type II Collagen⁺, osteocalcin⁻, type X collagen⁻ [86]; and hematopoietic precursors that are CD34⁺.

Step (f) will typically involve culturing the ES cell(s) in the presence of one or (usually) more growth factors, other polypeptides, vitamins, cations and/or hormones, *etc*. As an alternative to using exogenous growth factors *etc.*, differentiation can be achieved by inhibiting endogenous expression of certain genes, using techniques such as gene knockout, antisense, RNA silencing (*e.g.* RNAi [*e.g.* refs. 48-52]), *etc.*

ES cells may be cultured in the presence of components such as growth factors, transcription factors, hormones, metal ions, vitamins, steroids, and/or other organic compounds, *etc.* Specific components for use during ES cell culture include but are not limited to: tissue necrosis factor α (TNF-α); TNF-β; interferon α (IFN-α); IFN-β; IFN-γ; one or more interleukins (IL), including IL-1, IL-2, IL-3, IL-4, IL-5, IL-6, IL-7, IL-8, IL-9, IL-10, IL-11, IL-12, IL-13, IL-14, IL-15, IL-16, IL-17 & IL-18; fibroblast growth factors (FGF), including FGF-1 (or acidic FGF), FGF-2 (or basic FGF), FGF-3, FGF-4, FGF-5, FGF-6, FGF-7 (or keratinocyte growth factor), FGF-8, FGF-9, FGF-10, FGF-11 (or FHF-3), FGF-12 (or FHF-1), FGF-13 (or FHF-2), FGF-14 (or FHF-4), FGF-15, FGF-16, FGF-17, FGF-18, FGF-19, FGF-20, FGF-21, FGF-22 and FGF-23; transforming growth factor α (TGF-α); transforming growth factor β (TGF-β) 1 or 2, or a 1.2 heterodimer; bone morphogenic proteins (BMPs), including BMP-2 and BMP-4, and BMP-3, BMP-3B, BMP-5, BMP-6, BMP-7 and BMP-8; cartilage-derived morphogenetic proteins 1, 2 and 3 (growth/differentiation factors 5, 6 and 7); hepatocyte growth factor (HGF); epidermal growth factor (EGF); β nerve growth factor (NGF); neurotrophins, such as NT-3; brain-derived neurotrophic factor (BDNF); activin A; vascular endothelial cell growth factor (VEGF); granulocyte colony stimulating factor (GCSF); granulocyte macrophage colony stimulating factor (GMCSF); platelet-derived growth factor (PDGF); insulin; insulin-like growth factor I (IGF-1); IGF-2; erythropoietin (Epo); stem cell factor (SCF); glucagons; glucagons-like peptide 1 (GLP-1); Flt-3 ligand; hedgehog proteins, such as sonic hedgehog; Ca²⁺; Mg²⁺ ; pyruvate; butyrate; retinoic acid; fibroblast growth factor 2 (FGF-2); dimethylsulfoxide (DMSO); hexamethylene bisacetamide (HMBA); glucocorticoids, such as dexamethasone; extracellular matrix-promoting agents, such as ascorbic acid (ascorbate); cell extracts; hydrocortisone; catecholamines; epinephrine; mineralisation promoters, such as β-glycerophosphate; triiodothyronine; a demethylation reagent *e.g.* 5-aza-2'-deoxycytidine; serum albumins; antibiotics; antifungals; amphotericin-B; *etc.* These components will generally be included in or added to the medium in which the ES cells (or, as culture proceeds, their differentiation products) are being cultured. Suitable concentrations for differentiation can be determined routinely and empirically, but will typically fall in the range 1µM-1mM, or 1µg/ml-10mg/ml.

Other techniques for inducing differentiation include exposure to physical factors *e.g.* temperature, reduced or increased oxygen levels (*e.g.* hypoxia), reduced or increased CO₂ levels, reduced or increased pressure, microgravity [53] or acceleration, *etc*.

Various protocols for producing specific cell lineages and types from human ES cells have been disclosed in the art. These protocols may be used in step (f) of the invention, including but not limited to those summarised in the following table:

| **Cell type** | **Ref.** | **Summary of conditions** |
|---|---|---|
| Hepatocytes | 54, 55 | Culture in presence of n-butyrate. With or without feeder layer. Can proceed via or not via EBs. |
| Neural precursors | 41, 56 | Culture EBs with fibroblast growth factor 2. Withdrawal of FGF-2 resulted in differentiation to neurons, astrocytes and oligodendrocytes. See also ref. 57 to compare with ref. 59. |
| | 58 | Culture of EBs with mitogens. |
| | 59 | Culture in presence of EGF and bFGF. Not via EBs. |
| Neurons | 43 | Culture in presence of retinoic acid and NGF. |
| | 60, 61 | Transplantation into chick embryos. |
| | 43 | Culture with retinoic acid or β-NGF. |
| | 62 | Culture EBs with or without retinoic acid, then culture with EGF, FGF-2, PDGF and IGF-1. |
| | 63 | Transfection with vector encoding FGF-2. |
| Dopaminergic neurons | 62 | Culture EBs with retinoic acid, then culture with EGF, FGF-2, PDGF and IGF-1, then separate NCAM^{+ve} cells or A2B5^{+ve} cells and culture these with NT-3 and BDNF. |
| | 62 | Culture EBs with FGF-2, BDNF and NT-3, then culture with BDNF & NT-3 and/or cAMP & ascorbic acid. Use of Epo increases proportion of dopaminergic cells. |
| Neuronal precursors, glial cells, oligodendrocytes,. | 62 | Culture ES cells with TGF-β superfamily antagonists (*e.g.* noggin or follistatin). |
| Hematopoietic precursors | 64, 65 | Co-culture with stromal cell lines derived from mouse hematopoietic tissue (yolk sac or bone marrow). The precursor cells can then give rise to myeloid, erythroid and megakaryocyte colonies. |
| | 66 | Culture with methylcellulose. |
| | 67 | Culture EBs with stem cell factor (SCF), Flt-3 ligand, IL-3, IL-6 and G-CSF. Optional further culture with SCF, GM-CSF, IL-3 and Epo. BMP-4 also used. |
| | 68 | Treatment during EB development with cytokines and BMP-4. |
| Endothelial cells | 69 | Culture of EBs followed by immunoaffinity extraction of cells using anti-PECAM1 antibodies. |
| | 70 | Culture in medium containing VEGF, bFGF, IGF-1 &/or EGF. |
| Cardiomyocytes | 71 | Transfection of ES cells with constructs encoding HIV proteins tat and/or nef. |
| | 44 | Plating of EBs on gelatin-coated culture dishes. |
| | 72 | Plating of EBs on gelatin-coated plates or chamber slides. |
| | 73 | Culture EBs for 4 days in 80% KO-DMEM, 1mM L-glutamine, 0.1mM β-mercaptoethanol, 1% non-essential amino acids, 20% FBS, then transfer onto gelatin-coated or poly-L-lysine-coated plates, with optional use of 5-aza-dC. Cardiomyocytes then enriched using Percoll gradients. |
| | 74 | Treatment with ascorbic acid (vitamin C), not via EBs. |
| | 75 | Culture with extraembryonic tissue. |
| | 76 | Co-culture with murine visceral-endoderm-like cells. |
| Insulin secreting cells | 77 | Culture of EBs, or replating on gelatinised tissue culture plates in the absence of a feeder layer. |
| | 78 | Expression of *pdx-1* gene after removal of feeder cells. |
| | 79 | Culture EBs with: (1) retinoic acid, selenium & thyroid hormone receptor ligand T3; then (2) noggin, EGF &bFGF; then (3) nicotinamide. |
| Trophoblast | 80 | Culture with BMP-4. |
| Epithelium | 60, 61 | Transplantation into chick embryos. |
| Ectoderm | 81 | Culture in a non-agglutinated state. |
| Keratinocytes | 82 | Culture with BMP-4 and/or ascorbic acid. |
| Non-extraembryonic endodermal lineages | 83 | Culture in presence of BMP-2 pathway antagonists, such as noggin, fetuin, chordin, gremlin, follistatin, amnionless, cerberus, DAN, or the ectodomain of BMPRIA. |
| Muscle | 75 | Culture with extraembryonic tissue. |
| Osteogenic lineage | 84 | Adherent culture of EBs in presence of dexamethasone, ascorbic acid and β-glycerophosphate. |
| Osteoblast precursors and multipotent mesenchyma | 85 | Culture with a bone morphogenic protein, a ligand for the TGF-β receptor, and/or a ligand for the human vitamin D receptor. May also include dexamethasone, ascorbic acid-2-phosphate, Ca²⁺ and/or PO₄³⁻. |
| Chondrocytes and precursors | 86 | Culture EBs with one or more of transforming growth factor, fibroblast growth factor, growth and differentiation factor, hedgehog protein, bone morphogenic protein, L-ascorbic acid and/or parathyroid hormone-related protein. Can use micromass culture. |

With reference to this table, the first column lists desired cells of the invention and the third column summarises conditions for use in step (f), with detailed conditions (including concentrations, timings, etc.) being found in the references themselves. Thus the invention provides, for instance, a process for preparing a hepatocyte, comprising steps (a) to (f) as described above, wherein differentiation step (f) proceeds as described in one of the citations listed in the table. For example, step (f) may involve cell culture in the presence of n-butyrate, *etc.* As shown, however, there is often more than one *in vitro* route to the same cell type, and so the protocols cited in the table should not be seen as limiting the scope of the invention.

As described above, differentiation in step (f) will typically *occur in vitro.* As an alternative, differentiation can take place *in vivo,* such as in a host animal. For example, human ES cells can be injected into mice, where they develop and differentiate *in vivo* in a similar way to teratomas. The cells can then be extracted from the host and desired human cell types can be prepared.

When performed *in vitro,* step (f) may be performed in the presence or absence of serum. If serum is not used, a serum replacement may be used instead. Fresh medium may be supplied during step (f).

Reference 87 describes the co-culture of ES cells with "micro-organs" [88] to facilitate differentiation. These "micro-organs" are samples of biological organs which are of sufficient size to preserve the micro-architecture of the original tissue/organ, but which are small enough to allow efficient nutrient and waste exchange via diffusion with a liquid medium in which they are located. This technique may be used according to the present invention.

Differentiation of ES cells often gives rise to more than one cell type as a result of different differentiation rates, asymmetric cell division, differentiation factor secretion from cells as maturation progresses, *etc.* It is rare, therefore, that the cells obtained after step (f) will consist solely of the desired cell type. A population of cells including the desired cells is a more typical result. However, the desired cells can generally be separated from any unwanted cells by selection, by re-plating, by cell sorting (*e*.*g*. FACS [45]), *etc.*

Cells resulting from step (f) may be in the form of individual cells or in the form of aggregates. Where aggregates arise, these can be dis-aggregated (*e.g.* enzymatically), and the dis-aggregated cells may then re-aggregate. Dis- and re-aggregation in this way offers a way of removing unwanted cells within an aggregate (*e.g.* dead or undifferentiated cells). It also assists in cryopreservation as single cells are in general more able to survive cryopreservation than clumps of cells. Cells resulting from step (f) may also be in the form of tissues or organs.

ES cells of the invention can also be used to re-program other human cells (transdifferentiation) *e.g.* by co-culturing them as described in reference 89. Thus the invention provides a process for preparing a desired cell, comprising steps (a) to (e) as described above, and the further step of: (f) culturing a starting cell with an ES cell of the invention in order to transdifferentiate the starting cell into the desired cell. The invention also provides cells obtained and/or obtainable by this method.

### Therapeutic compositions and methods

Stem cells and their differentiation products can be used to treat disease. The cells may be terminally differentiated when delivered, or final stages of maturation might take *place in vivo* after delivery.

The invention therefore provides a method of therapy, comprising the step of delivering ES cells of the invention, and/or their differentiation products, to a patient. The invention also provides ES cells of the invention, and/or their differentiation products, for use as a medicament. The invention also provides the use of ES cells of the invention, and/or their differentiation products, in the manufacture of a medicament for treating a patient.

The patient will be suffering from, or be at risk of, a disease or condition susceptible to treatment by delivery of the cells. The patient will typically be of the same organism as the cells *e.g.* a human. The patient may be a child (*e.g.* a toddler or infant), a teenager or an adult. The patient may be unborn.

In general, the invention can be used to treat any disease that can benefit from the replacement or of damaged or missing cells, or modulation (*e.g.* by transdifferentiation) of *in situ* cells. Diseases and conditions susceptible to treatment by delivery/implantation of ES cells and/or cells differentiated from ES cells include, but are not limited to: diabetes (delivery of glucose-sensitive insulin-secreting cells); Parkinson's disease (dopamine-producing neurons [90] or naïve ES cells [91]); Huntington's disease (GABAergic neurons); spinal cord injury (motor neurons); amyotrophic lateral sclerosis (motor neurons); Alzheimer's disease (neural precursors); myocardial infarction, ischemic cardiac tissue or heart-failure (partially- or fully-differentiated cardiomyocytes [92,93,94]); side-effects of radiation *e.g.* in transplant patients (hematopoetic precursors); corneal scarring (corneal stem cells); liver cirrhosis or failure (hepatocytes); ischemic brain damage (neurons); spinal cord damage; cartilage damage (chondrocytes); bone damage (osteogenic lines, including osteocytes); osteoarthritis (chondrocytes); myelination disorders, such as Pelizaeus-Merzbacher disease, multiple sclerosis, adenoleukodystrophies, neuritis and neuropathies (olifodendrocytes); in bone marrow transplantation settings *e.g.* for treating bone marrow failure, leukaemia, lymphoma, myeloma, and other tumors (hematopoietic stem cells). Cells of the invention may also be used to prevent the development of scar tissue.

With a view to use in therapy, the invention also provides compositions comprising ES cells of the invention and/or their differentiation products. The pH of these compositions is preferably between 6 and 8, preferably about 7. The pH may be maintained by the use of a buffer. The compositions may be sterile and/or pyrogen-free. The compositions may be isotonic with respect to humans.

Compositions of the invention will typically, in addition to cells, comprise one or more 'pharmaceutically acceptable carriers', which include any carrier that does not itself induce the production of a response which is harmful to the patient receiving the composition. Suitable carriers are typically large, slowly metabolised macromolecules such as proteins, polysaccharides, polylactic acids, polyglycolic acids, polymeric amino acids, amino acid copolymers, and lipid aggregates (such as oil droplets or liposomes). Such carriers are well known to those of ordinary skill in the art. The compositions may also contain diluents, such as water, saline, glycerol, *etc. (e.g.* Ringer's solution, Krebs-Ringer solution, *etc.*). Additionally, auxiliary substances, such as wetting or emulsifying agents, pH buffering substances, and the like, may be present. A thorough discussion of pharmaceutically acceptable excipients and ingredients is available in reference 95, and reference 96 gives details on formulating cells for therapeutic delivery.

The compositions may also contain a preservative. The compositions may also contain one or more antibiotics and/or antimycotics. The compositions may also include a medium to maintain the cells (preferably serum-free, in order to avoid provoking an undesired immune response). The compositions may also include one or more anti-inflammatory agent, anticoagulants, etc.

Compositions of the invention will generally be administered directly to a patient. Direct delivery may be accomplished by injection (*e.g.* subcutaneously, intraperitoneally, intravenously, intramuscularly, or to the interstitial space of a tissue) or by simple application or administration. Delivery will usually be targeted to the anatomical region where the cells are found *in vivo.* For example, lung cells will be delivered to the lung, hepatocytes will be delivered to the liver, glucose-sensitive insulin-secreting cells will be delivered to the pancreas, lympocytes will be delivered to the lymphatic and/or blood system, *etc.*, although other routes are possible *e.g.* glucose-sensitive insulin-secreting cells can be delivered to the liver via the hepatic portal vein, where they can function, and cells to prevent scarring will be delivered at the site of injury.

The number of cells to be delivered to a patient may be based on a number of factors, including: the body weight of the recipient, the severity of the disease, the number of cells surviving within the subject, *etc*. Typically, the number of cells administered to a patient will be sufficient for the cells to bring about a physiological response. A typical number of cells would be around 10⁷ to 10⁸ cells per kg body weight, although fewer cells may be used *e.g.* if the recipient is tolerised to them. It may be necessary to repeat infusions of cells over several months to achieve a desired cumulative total mass.

Procedures for the preparation of cells of the invention for transplantation into a subject will follow the art applied to the known preparation of other cells transplantation. This includes methods of protecting cells against an inflammatory response by the transplant recipient. For instance, the cells of the invention may be co-administered with anti-inflammatory agent(s), or they may be masked from the immune system. The cells may be delivered in conjunction with an anticoagulant (*e.g.* heparin) and/or with human serum albumin (preferably recombinant), typically in the same injection. Cells may also be delivered in conjunction with immunomodulatory agents *e.g.* immunosuppressants or immunoadjuvants.

Cells may be encapsulated or surrounded. A typical surrounding semi-permeable membrane will permit the passage of soluble proteins, growth factors, hormones, *etc.*, but will not permit the passage of cells *(e.g.* T cells and B cells). Similarly, the cells may be encapsulated *e.g.* in an alginate hydrogel [97] or in any other biodegradable and biocompatible material (*e.g.* poly(lactide-co-glycolide) 'PLG', foam-like membranes [98], acrylic copolymers [99], aminopropyl-silicate membranes [100] *etc.).* Cells may be encapsulated in liposomes, including those which are targeted *e.g.* immunoliposomes.

Cells may also be transplanted with biocompatible and/or biodegradable scaffolds (*e.g.* collagen, fibrin, hyaluronic acid, proteoglycans, PLG, hyaluronan, keratin, *etc.*). Biomimetic scaffolds are cell growth supports used in tissue engineering techniques to provide a structural and mechanically sound three dimensional structure for promoting tissue *growth in vivo* [*e.g.* refs. 101-110]. They can take various forms *e.g.* foams, fibres, gels, membranes, *etc*. Scaffolds may be delivered to patients loaded with cells of interest. In an alternative arrangement, the stem cells themselves can form the scaffold, either in undifferentiated or differentiated form.

Cells of the invention may be matched (*e.g.* by HLA-typing) to the recipient patient in order to avoid rejection [111]. This can conveniently be achieved by preparing the cells from stem cells which are compatible with the recipient. It is also possible to use un-matched or partially-matched cells if a recipient has been pre-tolerised to the cells (*e.g.* by using tolerogenic antigen-presenting cells) or has been appropriately immunosuppressed.

Included within the invention are methods and uses in which immature cells are transplanted into a recipient, where they subsequently mature into cells of the invention. For example, Parkinson's disease may be treated by injecting ES cells which then differentiate *in vivo* into dopamine neurons, rather than injecting the cells which have already differentiated [91].

### Protein purification

Many cells secrete substances *in vivo* which are useful therapeutic products *e.g.* hormones such as insulin and oestrogen, interferons, interleukins, erythropoietin, neuropeptides, growth factors, *etc*. These substances may be secreted *in vivo* at low concentrations, and they may be difficult to purify from bodily sources. It is possible to prepare cells from bodily sources (*e.g.* from cadavers) and then purify the substances (*e.g.* insulin purified from bovine pancreas), but the preferred route is normally recombinant production (for polypeptides) or chemical synthesis (*e.g.* for steroids).

Differentiation from ES cells of the invention offers an alternative source of secretory cells which can be prepared in large numbers and high purity for polypeptide production. Such cells are essentially native and so offer advantages over recombinant production *e.g.* post-translational processing, such as glycosylation and oligomeric assembly, will be native.

The invention thus provides a process for preparing a secreted factor of interest, comprising steps (a) to (f) as defined above, and further comprising the steps of: (g) culturing the cells obtained in step (f) in a culture medium under conditions such that they produce the factor of interest and secrete it into the culture medium; and (h) purifying the factor from the culture medium. The invention also provides a factor obtained and/or obtainable by this method. Preferred factors are polypeptides *e.g.* hormones, growth factors, interleukins, interferons, *etc.*

The invention also provides ES cells derived from tripronucleated zygotes, for use in the expression and/or purification of secreted proteins.

The invention also provides a process for preparing nucleic acid from a cell, comprising steps (a) to (f) as described above, and further comprising the step either of: (g1) preparing genomic DNA from a cell obtained in step (f), or (g2) preparing mRNA from a cell obtained in step (f) and, optionally, preparing cDNA from said mRNA.

### Toxicology and metabolism testing

Prior to administration to humans, pharmaceuticals undergo various stages of testing, with safety (metabolism, biodistribution, toxicity, side-effects, *etc.)* being tested at an early stage in research and development. Other things which are subjected to similar testing regimes include cosmetics, prostheses, implants, *etc.* Human testing of these items is both difficult and expensive, and so tests are often performed on tissue cultures instead. However, access to adequate amounts of fresh tissue is limited, and the usual alternative cells, namely cell lines, do not represent normal tissue. Furthermore, it is difficult to find cells with a cross-section of genotypes and phenotypes which is broad enough to ensure that the test is representative of the population as a whole.

The ES cells of the invention can address these difficulties [58,112-115]. In particular, the invention can be used to prepare ES cells from a large cross-section of genotypes and phenotypes, thereby giving a better representation of a population. The ES cells themselves can also be used for testing toxic effects against embryonic cells [116].

Thus the invention provides a process for preparing a panel of cells, tissues or organs of different cell types, comprising steps (a) to (f) as defined above, wherein at least step (f) is performed at least twice, thereby providing the panel. Thus a panel of different cell types can be obtained from the same ES cell population (*i.e.* ultimately from the same tripronucleated zygote), and/or from different ES cell populations (*i.e.* from different tripronucleated zygotes), and substances can be tested on these different cell types. Step (a) may therefore be performed one or more times in the overall process.

The panel is suitable for testing a variety of substances for their effects. For example, they can be used for assessing toxic effects, metabolism, allergic reactions, side effects, biodistribution, inflammatory reactions, contact reactions, dermatological effects, *etc*. Examples of substances for testing include pharmaceuticals, cosmetics, foodstuffs, hygiene products (particularly paper products, such as diapers, catamenials, tampons, *etc.*), clothing materials, prostheses, surgical implants, and natural products. The effects of radiation can also be tested.

A typical panel will comprise one or more of the following cell types: hepatocytes; comeocytes; corneal tissue; epidermis; keratinocytes; fibroblasts; kidney cells; lung tissue; pneumocytes; lymphocytes; neurons; testis; and ovary.

For cosmetic and dermatological testing, a panel of epithelial cells is particularly useful. Corneal and conjunctival cells are also useful for cosmetic testing. For allergy testing, IgE-secreting lymphocytes are particularly useful. The invention is particularly useful for studying the effect of substances on the liver (*e.g.* for toxicological testing) and hepatocytes are thus preferred cells for the panels. The invention allows the production of a consistent source of essentially-normal human liver tissue for toxicology studies.

The invention also provides a process for preparing a panel of cells of the same cell type, comprising at least two separate runs of steps (a) to (f) as defined above, wherein each run uses a different tripronucleated zygote in step (a). Preferably different runs yield the same cell type but from different starting material, such that a population of a single cell type (*e.g.* a population of hepatocytes) can be prepared. The population might be designed, for example, to contain major cytochrome P450 (CYP) alleles *e.g.* CYP2A5, CYP2A6, CYP2D6, CYP2C9, CYP2C19, CYP3A4, *etc.*

The invention also provides a process for testing the effects of a test material, comprising preparing a panel of cells of the same or different cell type, as disclosed above, and further comprising the steps of: (g) contacting the cells obtained from step (f) with the test material; and (h) detecting any effects on the cells arising from step (g).

Metabolism and/or toxicological effects can be assessed in standard ways *e.g.* cell death, morphological changes, shock responses, membrane permeability, mutagenic studies, toxicogenomics, *etc.*

The test material may be a pharmaceutical (or a mixture, to determine the effect of interactions), a cosmetic, a foodstuff, a hygiene product, a clothing material, a prosthesis, a surgical implant (or any of the same at a preliminary or candidate stage), *etc.*

The invention also provides ES cells derived from tripronucleated zygotes, for use in assessing toxic effects, metabolism, allergic reactions, side effects, biodistribution, inflammatory reactions, contact reactions, and/or dermatological effects of a test material. Use in toxicology and/or metabolism testing is preferred.

### Screening methods

Cells of the invention can be used *in in vitro* screening assays *e.g.* to identify compounds which have a pharmacological effect on the cells. The invention therefore provides an *in vitro* screening assay, comprising steps (a) to (f) as described above, and further comprising steps: (g) contacting the cell obtained in step (f) with a test compound; and (h) detecting in a cell the presence or absence of a change arising from step (g).

Typical test compounds include, but are not restricted to, peptides, peptoids, proteins, lipids, metals, nucleotides, nucleosides, small organic molecules, antibiotics, polyamines, and combinations and derivatives thereof. Small organic molecules have a molecular weight of more than 50 and less than about 2,500 daltons, and most preferably between about 300 and about 800 daltons. Complex mixtures of substances, such as extracts containing natural products, or the products of mixed combinatorial syntheses, can also be tested and the component that causes secretion can be purified from the mixture in a subsequent step.

Test compounds may be derived from large libraries of synthetic or natural compounds. For instance, synthetic compound libraries are commercially available from Maybridge Chemical Co. (Trevillet, Cornwall, UK) or Aldrich (Milwaukee, WI). Alternatively, libraries of natural compounds in the form of bacterial, fungal, plant and animal extracts may be used. Additionally, test compounds may be synthetically produced using combinatorial chemistry either as individual compounds or as mixtures.

The screening method may be in a high-throughput format. Preferably, all the biochemical steps for this assay are performed in a single solution in, for instance, a test tube or microtitre plate, and the test compounds are analysed initially at a single compound concentration. For the purposes of high throughput screening, the experimental conditions are adjusted to achieve a proportion of test compounds identified as "positive" compounds from amongst the total compounds screened. The assay is preferably set to identify compounds with an appreciable affinity towards the target *e.g.*, when 0.1% to 1% of the total test compounds from a large compound library are shown to bind to a given target with a Kᵢ of 10µM or less (*e.g.* 1µM, 100nM, 10nM, or tighter).

The change in step (h) may, for instance, involve the morphology, marker phenotype, or functional activity of the cells being used. By comparing these characteristics to untreated control cells, the change can be attributed to the test compound.

General guidance on these assays can be found in reference 117.

The invention also provides a compound identified by the method of the invention, optionally in combination with a pharmaceutical carrier. The compounds may themselves be useful pharmaceuticals or they may be useful lead compounds for development into pharmaceuticals.

Screening assays can also be used to identify factors or conditions which can be used for inducing ES cell differentiation. Thus the invention provides *an in vitro* screening assay, comprising steps (a) to (e) as described above, and further comprising steps: (f) contacting the ES cell obtained in step (e) with a test compound and/or exposing the ES cell to altered environmental conditions; and (g) detecting in a cell the presence or absence of differentiation. Typical test compounds include those mentioned above, and also the various growth factors, transcription factors, hormones, metal ions, vitamins, steroids, and/or other organic compounds, etc. mentioned above.

### Genetic modification

Like any other type of ES cell, the ES cells of the invention are suitable for genetic modification. Thus the invention provides a process for preparing a genetically-modified ES cell, comprising steps (a) to (e) as described above, and further comprising the step: (f) genetically modifying the ES cell.

Techniques for genetic modification of ES cells are well known in the art [118]. Genetic manipulation may involve activation or over-expression of endogenous genes and/or introduction of exogenous genes. In general, step (f) will involve transfection of the ES cells with a vector. Viral and non-viral vectors can be used. The use of viral vectors (especially lentiviral vectors) is described in refs. 119-121.

The use of suicide genes provides a method of selectively killing cells which may persist in cell preparations to be administered to patients (*e.g.* undifferentiated stem cells), or all cells (differentiated or undifferentiated) derived from the stem cells as a failsafe mechanism to destroy the cells after transplantation. Suicide genes encode protein products that have no appreciable direct effect on cellular function, but which are capable of conferring toxicity by their ability to convert otherwise non-toxic substances (frequently termed prodrugs) into toxic metabolites. Suicide gene technology has been developed as a means of rendering cancer cells more sensitive to chemotherapeutics and also as a safety feature of retroviral gene therapy. Several combinations of suicide genes and prodrugs are known in the art [*e.g.* ref. 122] and include: *E.coli* cytosine deaminase + 5-fluorocytosine; HSV thymidine kinase + ganciclovir or acyclovir; *E.coli* nitroreductase + CB1954, *etc.* The suicide gene is preferably under the control of a promoter expressed in undifferentiated stem cells or in other cells undesirable for transplantation (*e.g.* tumors or tumorigenic cells), in which case undifferentiated cells can be removed from culture by using the appropriate prodrug without affecting differentiated cells. For use as a failsafe mechanism to allow selective killing of a transplant in a patient (*e.g.* where the transplant is found to be harmful in a recipient), however, the suicide gene will generally be under the control of a constitutive promoter, although tissue-specific or inducible promoters may be used. The introduction and use of suicide gene vectors in ES cells is described in ref. 123.

Cells may be genetically manipulated to encode a polypeptide (*e.g.* a transcription factor) which promotes differentiation of the stem cell into a desired cell. Expression of this polypeptide may be controlled so that it occurs in the stem cell itself, or so that it occurs in a derivative of the stem cell *(e.g.* in EBs). Conversely, a cell may have been genetically manipulated such that it under-expresses or does not express a polypeptide (*e*.*g*. a transcription factor) which either favours differentiation of the stem cell away from a desired phenotype or which inhibits differentiation into a desired cell

A cell may have been genetically manipulated to insert markers suitable for lineage selection, a technique which specifically selects a desired cell type *e.g.* based on a previously-inserted recombinant construct which comprises a tissue-specific promoter linked to a selectable marker. Where the efficiency of differentiation of stem cells into cells of the invention is inherently low, this technique allows efficiency to be increased. Suitable selectable marker genes include, but are not restricted to, drug selectable genes (*e.g.* the G418 resistance gene, zeo, bsd, HPRT, hygro, puro), visible markers such as fluorescent proteins (*e.g.* GFP, DsRed) and genes which facilitate selection by automated cell sorting (*e.g.* genes encoding cell surface antigens). Genetically-modified lineage-defective ES cells are described in ref. 124.

A cell may have been genetically manipulated to insert markers suitable for enrichment of cells from within a heterogeneous population. One possibility is to place a marker protein under the control of a promoter that is specific to a desired cell type such that the marker is transcribed in parallel to natural cell-type-specific genes. The marker can then be detected and used for enriching cells of the invention (*e.g.* by FACS).

A cell may have been genetically manipulated so that expression of reactive antigens is reduced or eliminated. For instance, genes which encode auto-antigens could be knocked out, or they could be inhibited using antisense techniques or RNA silencing (*e.g.* RNAi).

The genetic manipulations described above may be used singly, or two or more may be used in combination.

Genetic manipulation of the cell may occur through random integration into the genome or by gene targeting. As an alternative the manipulation may, where appropriate, use an episomally-maintained vector (*e.g.* a plasmid).

For random integration, vector(s) which encode the relevant polypeptides may be introduced into the cell. Typically, an expression vector comprising a gene promoter operably linked to DNA encoding the relevant polypeptide would be used. The coding DNA may be cDNA, genomic sequences or a mixture of both. The promoter may direct constitutive or inducible expression and may be tissue-specific. Examples of constitutive promoters include the promoters from β-actin, phosphoglycerate kinase (PGK), glycolytic enzymes, elongation factor 1α (EF1α), or SV40. Examples of inducible gene promoters include systems composed of a chimeric transactivator that reversibly binds to the promoter region of the expression construct in response to a drug or ligand (*e.g.* doxycycline, ecdysone, mifepristone, tetracycline, FK1012, or rapamycin).

An alternative to random integration is the precise alteration of genes *in situ* by homologous recombination, termed "gene targeting". This is the precise predetermined modification of genes by homologous recombination between exogenous and endogenous DNA. Gene targeting can be used to insert, replace, rearrange or remove chosen DNA sequences in cultured cells, most commonly embryonic stem cells [*e.g.* chapter 3 of reference 7, and refs. 125 & 126]. In some circumstances gene targeting may be preferable to simple introduction of an expression vector at a random site because the genetic modification can be predetermined to avoid any deleterious effect (*e.g.* oncogenic transformation) that would reduce the therapeutic value of derived cells. Homologous recombination techniques for human ES cells are also disclosed in ref. 127.

Gene targeting may be used to achieve constitutive or inducible expression of a gene of interest by modifying or replacing the natural promoter or other regulatory regions of that gene. For example, a gene promoter may by replaced with a constitutive or inducible promoter or elements which direct constitutive expression may added adjacent to the endogenous gene promoter.

Methods to achieve such modifications by gene targeting, including in ES cells, are well known in the art.

The generation of knockout ES cell mutants is disclosed in chapter 16 of ref. 7.

It is also possible to perform genetic manipulation on a cell other than a stem cell, and then to transfer that genetic manipulation into a stem cell (*e.g.* by transfer of a nucleus into an enucleated stem cell) or into an cell which can give rise to a stem cell (*e.g.* by transfer of a nucleus into an enucleated oocyte). Both of these approaches indirectly give a genetically-manipulated stem cell.

Stem cells of the invention may also be used as donors or acceptors for nuclear transfer.

### General

Cells and processes of the invention may be located or performed *in vitro* or *in vivo.*

Cells may be cryopreserved at any stage *i.e.* before and/or after differentiation. Suitable cryopreservants are well known in the art [*e.g.* see ref. 128]. For cryopreservation of ES cells, aggregates of cells are typically used.

The term "comprising" can mean "including" as well as "consisting of" *e.g.* a composition "comprising" X may consist exclusively of X or may include something additional *e.g.* X + Y.

The term "about" in relation to a numerical value *x* means, for example, x±10%.

The word "substantially" does not exclude "completely" *e.g.* a composition which is "substantially free" from Y may be completely free from Y. Where necessary, the word "substantially" may be omitted from the definition of the invention.

A conceptus is considered a zygote from conception to day 14, an embryo from day 15 to day 56, and a fetus from day 57 to birth.

### BRIEF DESCRIPTION OF THE DRAWINGS

Figure 1 illustrates the method of the invention for producing ES cells from triploid zygotes.

### MODES FOR CARRYING OUT THE INVENTION

### Blastocyst production

Tripronuclear human zygotes were obtained for micromanipulation or for culture on day 1 of a conventional IVF programme (day zero being oocyte retrieval). The use of abnormally-fertilized human zygotes for research was approved by the institutional review board on the use of human subjects in research at the Instituto Valenciano de Infertilidad (IVI), and complies with the Spanish Law of Assisted Reproductive Technologies (35/88). All patients participating in this study signed a written statement of consent and received information.

114 tri-pronucleated (3-PN) zygotes were obtained, each confirmed as having three pronuclei 16-18 hours after insemination. Protocols for follicular stimulation, oocyte recovery, *in vitro* insemination (day 0) and zygote culture (days 1 to 6) are described elsewhere [129].

For micromanipulation, 3-PN zygotes were placed in a small (25µl) drop of IVF-20 medium (MediCult, EMB, Barcelona, Spain) containing 7.5 µg/ml cytochalasin B (C6762; Sigma, Madrid, Spain) and 0.1 M demecolcine (D1925; Sigma, Madrid, Spain) and covered with equilibrated mineral oil. Zygotes were incubated for 2 minutes under controlled culture conditions (see below) and plates were then placed on the stage of an inverted microscope fitted with hydraulic micromanipulators (MM188, Narishige). Two microtools were used for micromanipulation: a holding pipette (100µm outer diameter; 19µm inner diameter); and a 6µm outer diameter opened microneedle for pseudonucleus aspiration. To accommodate the micromanipulation setup, both microtools were bent to a 45° angle and were used with a standard syringe system (IM-26-2; Narishige). Teflon tubing for hydraulic systems was filled with light mineral oil.

Before epronucleation, 3-PN zygotes were clumped against the holding pipette and rotated and repositioned until in a view. This rotation allows identification of all pronuclei and the two polar bodies. The three pronuclei were usually seen in a typical triangular fashion in the centre of the ooplasm in the same plane. At this stage, the supernumerary male pronucleus to be removed was at the farthest position from the second polar body [18, 19, 130, 131].

For epronucleation, the tip of the micropipette was inserted through the zona pellucida into the vitellus and gently positioned next to the surface of the targeted pronucleus. Negative pressure was then applied until the pronucleus was picked up. Pressure was maintained while the pipette and pronucleus were gently withdrawn from the syngamy area to the oolema. At this region, aspiration progresses up to total elimination of the pronucleus, with a small amount of cytoplasm being drawn freely into the epronucleation needle. The micropipette was then removed and the zygote was released. Each zygote manipulation took approximately 5 minutes.

After manipulation, zygotes were washed three times in IVF-20 medium and cultured in a 5% CO₂ atmosphere in air at 37.5°C for between 30 and 60 minutes. Zygotes were then assessed for survival and for their pronucleus count. Only surviving zygotes having two pronuclei went forward for culturing; degenerated zygotes or those not having two pronuclei were removed from culture.

Some tripronucleated zygotes were handled in the same way, except that they were not microsurgically manipulated. These zygotes served as a 3-PN control group.

Of 72 tripronucleated human zygotes, 56 (78%) survived microsurgical manipulation with two pronuclei. Normal general appearance of the zygotes was usually seen less than 30 minutes after manipulation, with the pronuclei being identifiable. At this time, some degenerative zygotes were also observed (n=16).

Rescued bipronucleated (2-PN) human zygotes and some 3-PN control zygotes were co-cultured until blastocyst stage on human endometrial epithelial cells (hEEC) [20]. 48 hours after insemination, two-to-four cell zygotes were grown in 1 ml of a 1:1 (v/v) mixture of IVF-20 and CCM (MediCult, EMB, Barcelona, Spain) until they reached the eight-cell stage. On the morning of day 3, cell masses were individually co-cultured on the hEEC monolayer and medium was replaced by 1 ml CCM. They were then cultured for three more days, with development being monitored each day.

During these experiments, developmental data from normal diploid zygotes obtained by *in vitro* insemination and co-culture in ongoing treatments were retrospectively and randomly grouped and served as a co-culture control group.

Developmental abilities of bi- and tri-pronucleated zygotes were statistically compared using a Chi-square test. When a single degree of freedom was involved, the Yate's correction for continuity was used.

After 24 hours of culture, all microsurgically-diploidised zygotes cleaved, and 96% reached the 6-8-cell stage at day 3 of development. On day 6, 21 rescued human zygotes progressed to the blastocyst stage (41%). The ability of diploidised zygotes to develop is comparable to the development of co-cultured normal fertilized zygotes (8-cell stage: 85%; blastocyst: 50%). Moreover, morphologically-normal blastocysts derived from epronucleated or co-cultured control zygotes were characterized by a well-defined ICM and trophoectoderm. In contrast, control 3-PN zygotes progressed significantly less efficiently in the co-culture system, with only 19% giving blastocysts. These were morphologically abnormal, characterized by an absent or reduced ICM and a linear trophoectoderm.

On day 5 or 6, eight morphologically-normal blastocysts derived from co-culturing of diploidised rescued zygotes were fixed and processed for assessing the presence of X and Y chromosomes and 18 autosomes (CEP 18 / X Orange / Y Aqua; Vysis Inc.) in at least 20 cells by triple FISH [132]. Four blastocysts derived from co-cultured 3-PN zygotes were similarly processed.

On Day 5, diploidised human blastocysts were placed in 0.2 ml PCR tubes containing 15 µl lysis buffer solution (50mM KCl, 10mM Tris-HCl pH 8.3, 0.5% Tween-20 and 100 µg/ml proteinase K). Every blastocyst was identified and immediately frozen and stored at -80°C until the PCR amplification.

Whole blood samples from zygotes' parents were collected in 5 ml EDTA tubes. The samples were identified and either directly frozen at -80°C or processed to obtain buffy coat specimens. Samples were then stored frozen at -15 to -25°C until DNA extraction using the PUREGENE™ genomic DNA isolation kit (Gentra Systems, Minneapolis, MN). Recovered genomic DNA was dissolved in a buffered solution containing a DNA stabilizer and stored at -20°C until PCR amplification.

PCR amplification of short tandem repeats (STRs) loci was performed using the AmpFLSTR™ Profiler Plus™ PCR amplification kit (Applied Biosystems, Foster City, CA). This kit co-amplifies ten polymorphic repeat regions and a segment of the X-Y homologous gene amelogenin.

The concentrations of PCR components and cycle parameters were as recommended by the manufacturer. To optimise results, 1.5 ng DNA input from blood samples was used. Before adding the PCR component mix, frozen blastocysts were thawed on ice for 2-3 minutes, followed by incubation at 37°C for 10 minutes, and then immediately placed at 95°C for 5 minutes.

For each locus, a mixture of 1.5 µl fluorescent PCR products, 12 µl deionised formamide and 0.5 µl size standard (GeneScan-500 [ROX], Applied Biosystems) was prepared and denatured at 95°C for 3 minute. The internal size standard loaded with each sample allowed automatic sizing of PCR products and normalised differences in electrophoretic mobility among samples.

Denatured samples were subjected to capillary electrophoresis using POP-4™ polymer, with 3 seconds of injection time. STRs data obtained from DNA amplification of human blood and diploidised cells were analysed using GeneScan™ Analysis Software 3.1.2 (Applied Biosystems). Three fluorescent dyes (blue 5-FAM, green JOE and yellow NED) were used with the kit for multicomponent analysis of samples. A fourth red dye (ROX) was used for internal size standards.

Using the four fluorescent dyes, detection and identification of positive control DNA fragments sized from 103 to 312 bp. Blood samples showed expected amplification patterns for the analysed STRs loci. In these samples, the fragments ranged from 103.24±0.07 (mean±SD) to 240.26 bp, which corresponds to amelogenin X, and the largest human alpha fibrinogen (FGA) locus, respectively.

Four of six analysed blastocysts (67%) showed a clear amplification pattern ranging from 103.93±0.53 (mean±SD) to 304.26 bp, corresponding to the amelogenin X gene and to the largest D18S51 allele, respectively. Analysis of the inheritance of the markers indicated that all four amplified blastocysts were diploid and genetically heteroparental. Three were female and one was male.

Thus the microsurgical epronucleation of tri-pronuclear zygotes according to the invention renders morphologically- and genetically-normal (diploid and heteroparental) blastocysts. Technically, the micromanipulation procedure results in successful survival rates and pronuclear number reconstruction, as 71% of manipulated zygotes survived and contained two pronuclei.

The survival rate, obtained without optimisation of techniques, is high enough for widespread application. Cytoskeletal relaxants (*e.g.* cytochalasin and colcemid) are routinely used to improve survival rates in mammalian nuclear transfer studies [133-135], as they induce the depolymerisation of actin filaments with possible effects on the plasma membrane. Even so, these drugs have been applied with caution on humans because of suggested cytotoxic effects. For instance, 30 minutes of treatment with cytochalasin types B (CCB) and D during microsurgical epronucleation of 3-PN zygotes impaired subsequent development [136,137]. However, limiting CCB and demecolcine exposure to a short 10 minute incubation rendered rescued zygotes able to cleave and develop to the blastocyst stage (41%) as efficiently as normally-fertilized controls. Effects of the cytochalasin type and treatment application (dose and time exposure) thus affect not only survival efficiency but also the subsequent developmental ability.

The handling control group was not an optimal selection, as 3-PN zygotes *in vitro* development progress is poor [138-140], finally rendering abnormal blastocysts having a reduced or absent ICM and linear trophoectoderm. These observations suggest that *in vitro* human embryonic development could be severely impaired by ploidy or possibly by parental combination, not being informative enough about CCB and demecolcine effects.

Concerning the relevance of ploidy or parental combination, dispermic 3-PN human zygotes are triploid and have the sex chromosome ratio XXX:XXY:XYY as 7:9:5 [141,142]. In all analysed tripronucleated-derived blastocysts derived in the present work, the distribution was altered to the 69,XXY karyotype, with no 69,XXX or 69,XYY cells being detected. This shift could indicate that, at more advanced stages, more limitations on development emerge according to the final parental combination, or might also suggest the need of a biallelic heritance for successful embryonic development. However, partial hydatidiform moles, typically having a triploid (69,XXX, 69,XXY and 69,XYY) karyotype and usually containing one maternal and two paternal complements [143], are able to implant [144]. It may thus be possible that 3-PN zygotes have different nutritional requirements, which are not supplied by the co-culture [139] or defined culture conditions [138,140].

Removal of the pronucleus with the farthest relative position from the 2PB restores the normal parental contribution, as suggested by the morphological and genetic observations, which confirms the paternal origin of such a pronucleus.

Rescued zygotes progressed in development, following the normal schedule and rendering 46,XX and 46,XY blastocysts (41%) containing well-defined inner cell mass and trophoectoderm. These results contrast with earlier reports where diploidized zygotes developed only to the 8-cell stage [131] and rarely to compaction stages [18,137]. As discussed above, the improvement achieved herein could be due in part to the use of cytoskeletal relaxants during manipulation, as well as to the epronucleation procedure itself.

When tri-pronucleated zygotes are dispermic in origin, a double paternal centrosome heritance would be expected [131,140]. Restoring the normal parental condition in polypronuclear zygotes by aspiration of the surplus pronucleus leaves the pronuclear membrane, juxta- and extranuclear components, including the extra centrosome [131], in the zygote, which could be the cause of observed impaired development. Alternatively, the pronuclear removal into a karyoplast could also eliminate those extra compounds, allowing subsequent normal embryonic development, as observed in mice [145,146]. Epronucleation as described herein removes the whole pronucleus and some amount of nearby cytoplasm, and normal *in vitro* embryonic development was observed.

As each locus is an independent marker, parental inheritance can be confirmed from those amplified loci from blood and blastocyst samples. DNA sample amplification can be improved by adding to lysis buffer bovine serum albumin to prevent or minimize the inhibition of PCR [147] by hemo compounds [148].

In a second series of experiments, again using cytoskeletal relaxants, X 3-PN zygotes were used, and X of these (X%) developed into diploid blastocysts. In a third series, this time avoiding cytoskeletal relaxants and instead using the method disclosed in reference 17, X 3-PN zygotes were used, and X of these (X%) developed into diploid blastocysts.¹
1 Waiting for data.

In conclusion, normal heteroparental human cells can be rescued from tri-pronucleated zygotes by removal of the surplus paternal pronucleus located at the farthest position to the second polar body. The 2PB second polar body can thus be considered as a good morphological indicator of the parental origin of the pronucleus.

### Preparation of blastocysts for ES cell production

Warmed diploidised blastocysts were cultured in 1 ml CCM in a 5% CO₂ atmosphere in air and 95% humidity at 37.5° for 12 hours to evaluate their survival and progression on development. Inner cell mass isolation was performed following an owner immunosurgical procedure. Thus, first of all the zona pellucida is removed by incubation in 1ml acidic Tyrodes' solution (ZD-10; Vitrolife) at room temperature for less than one minute. Zona-free blastocysts were washed twice in 1ml CCM and incubated in 1ml CCM under culture conditions for 3 hours. Then, zona free-blastocysts were incubated in 75µl drops of rabbit anti-human lymphocyte serum (CL8010, Cedarlane Laboratories Limited) in CCM (3:1, respectively) under mineral oil in culture conditions for 30 minutes. Then, blastocysts were briefly washed twice in 100µl CCM and incubated in 75µl of rabbit complement for use with monoclonal antibodies to human lymphos (Sera-Lab) in culture condition for 30 minutes. Blastocysts were then transferred to fresh 100µl CCM drop and gently pipetted to remove the lysed trophoectodermal cells. The isolated inner cell mass was then washed in 1 mL CCM for 30 minutes, being ready to be cultured on inactivated human endometrial stromal feeders to obtain human embryonic stem cells.

Blastocysts were also processed without immunosurgery. The zona pellucida was removed by incubation in 1 ml acidic Tyrodes' solution (ZD-10; Vitrolife) at room temperature for less than a minute. Zona-free blastocysts were washed twice in 1 ml CCM and incubated in 1 ml CCM under culture conditions for 3 hours. Zona-free blastocysts were then transferred to a human endometrial stromal feeder.

### Preparation of human feeder layers for ES cell derivation

Endometrial biopsies were obtained with consent from endometrial donors with a catheter (Gynetics, Amsterdam, The Netherlands) during the luteal phase, typically two days after hCG administration. Endometrial samples were placed in a drop of DMEM and cleaned with a scalpel, eliminating any clots and mucus. Then, using two scalpels in a scissor-like action, the tissue was cut into small pieces measuring less than 1mm. Once cut, the tissue was transferred to a conical tube containing 10 ml of a 0.1% collagenase IA solution (obtained by mixing 9 ml DMEM with 1 ml collagenase). To carry out the digestion, the Falcon tube was placed horizontally in a shaking water bath at 37°C for 1 hour. At the end of the incubation, the conical tube was removed, placed in a rack and left to rest for 10 minutes inside a laminar flow cabinet. The supernatant, in which the stromal cells are suspended, was collected and filtered under vacuum through a 30 µm pore-diameter membrane that had previously been sterilized by exposure to UV for 24 hours. The pellet was washed 3 times as follows: add 3-5 ml DMEM to the pellet, agitate to resuspend the cells, leave to rest for 5 minutes, and repeat the filtration procedure as before. The washed pellet was centrifuged at 2000 rpm for 10 minutes and the supernatant was discarded. The pellet contains stromal and blood cells, and was resuspended with an automatic pipette in 300 µl of a solution containing 4mg/ml DNAse. The reaction was stopped by adding 1 ml of 1% FBS in 9ml DMEM. Cells were counted to ensure a suitable seeding density of stromal cells (3×10⁶ per 5 ml). The medium was changed daily during early stages of culture (up to passage 2) and every two days thereafter.

When the cells reached 70-80% confluence, they were able to be passaged by washing them twice for 5 minutes each with Hanks' Balanced Salt Solution (HBSS). 3 ml trypsin-EDTA (1X) was added and the mixture was incubated for 5 minutes at 37°C. Cells were checked under a microsope to ensure that they had lifted off the plastic and entered suspension. If not, cells were dislodged by tapping the sides of the flask briskly. Trypsin was inactivated by adding 5 ml culture medium. The cell suspension was collected with a serological pipette, transferred to a 14 ml Falcon tube, and sealed with Parafilm. The cells were then centrifuged at 2000 rpm for 10 minutes and the supernatant was discarded, followed by resuspension in 2 ml culture medium. A 100 µl sample was removed for estimating the cell population.

Human endometrial stromal cells after passage three were inactivated by gamma irradiation to prepare a feeder layer that supports ES cell derivation. Cells in suspension were collected in 10 ml tubes and sealed with parafilm. The tubes were transported to an electron accelerator in a portable incubator at 37°C and 5% CO₂. Once in the accelerator, tubes containing the cells to be irradiated were placed on pieces of plasticine inside the tray and the tray was filled with 1.5 litres of sterile water at 37°C. The accelerator was programmed and cells were irradiated at 12 Gy. The cells were replaced in the portable incubator and returned to the laboratory. The cells were then seeded in a flask in culture medium containing 10% FBS and, after 24-48 hours, they were ready for use. The cells could also be stored for future use by freezing.

Irradiated human endometrial stromal cells were seeded into 6-well plates, as described above, at a density of 500,000 cells per well using stromal-cell culture medium (see below). Cells were incubated at 37°C in an incubator with 5% CO₂ for at least two days, with media being changed every 1-2 days. Conditioned media removed during this phase were centrifuged for 10 minutes at 2000 rpm to remove dead cells and were then retained for future use. When the feeders were ready to receive ICM cells, the medium was changed to hES medium (see below) supplemented with 4 ng/ml bFGF. Inner cell mass cells or zona free blastocysts were then added to each plate with hES medium.

### Passaging ES cells

The first passage was undertaken at the moment at which hES cells growing from the ICM filled the whole field of view of a 10X objective, and cells were always passaged at a ratio of 1:2. A Pasteur pipette with a diameter approximately equal to that of a day 5-day 6 blastocyst was used to mark a circle to define the outline of the population. A cross was marked in the centre of this circle to divide the colony into four sub-populations. Each sub-population was gently detached, either directly or by moving the plate inside the incubator so that the colonies could not aggregate. Plates were left in the incubator for 48 hours without removing them, followed by continued observation with medium being changed on day 3 or 4.

For the second passage, a new mechanical dispersion had to be performed by re-plating the newly-formed colonies at a ratio of 1:3. The same treatment as used for the first passage was used again, with dissected colonies being transferred to the new feeder layer with a Pasteur pipette. Plates were left to rest in the incubator for 48 hours, and the medium was changed for conditioned medium after 3-4 days.

For the third and subsequent passages, enzymatic dispersion was used. Cells were incubated for 6 to 10 minutes with type IV collagenase at 37°C. Cells were then aspirated with a pipette and moved up and down to remove the colonies. These cells were then transferred to new feeder plates that had previously been prepared.

### Culture media

500ml of stromal-cell culture medium² is prepared by mixing 300 ml DMEM, 100 ml MCDB-105 (Sigma), 40 ml fetal bovine serum (FBS), 220 µl insulin, 880 µl gentamycin and 880 µl fungizone (Gibco 15290-018). The mixture is then sterile-filtered (0.22µm) and stored at 4°C.
² This paragraph is subject to change.

The MCDB-105 for this medium was prepared as follows: (a) pour 800 ml embryo-transfer water (Sigma, W-1503) into a flask; (b) add the medium powder and dissolve by stirring to produce a yellow solution; (c) wash the original container with a little non-pyrogenic water to collect any remaining medium, and add this to the solution; (d) measure the pH, which should be between 4.9 and 5.1; (e) adjust pH to 7.1-7.2 with 0.5N NaOH; (f) adjust the volume to 1 litre with non-pyrogenic water; (g) filter through a 0.22*µ*m pore size membrane; (h) store in the dark at 4°C.

The FBS was prepared as follows: (a) warm the serum in a water bath at 56°C for 30 minutes; (b) cool to room temperature; (c) prepare 40ml aliquots; (d) store at -20°C.

The fungizone solution was prepared by dissolving the container-full in 20 ml embryo-transfer water.

The hES medium was prepared by mixing 10 ml KO-SR (Gibco 10828-028), 500 µl non-essential amino acids (Gibco MEM 100X, 11140-035), 50 µl β-mercaptoethanol, 250 µl of 200mM L-glutamine, and then KO-DMEM (Gibco 10829-018) up to a final volume of 50 ml. The final mixture was then filtered through a 0.22µm pore size membrane.

It will be understood that the invention has been described by way of example only and modifications may be made whilst remaining within the scope and spirit of the invention.

### REFERENCES (the contents of which are hereby incorporated by reference)

[1] National Institutes of Health, Dept of Health and Human Services Report. *Stem cells: scientific progress and future research directions.* June 2001 (*www.nih.gov*/*news*/*stemcell*/*scireport.htm*)*.*
[2] *Human embryonic stem cells* (2003) eds. Chiu & Rao. ISBN: 1588293114.
[3] Thomson *et al.* (1998) *Science* 282, 1145-7.
[4] Wobus (2001) *Mol Aspects Med* 22:149-64.
[5] Brivanlou *et al.* (2003) *Science* 300:913-915.
[6] Pera *et al.* (2000) *J Cell Sci* 113:5-10.
[7] Tymms & Kola (2001) *Gene Knockout Protocols* (Volume 158 of *Methods in Molecular Biology).* ISBN 1-59259-220-1. In particular see chapters 2, 3, 4, 16, 17 & 18.
*[8] Embryonic Stem Cells: Methods and Protocols* (ed. Turksen) 2001. ISBN 0896038815.
[9] Kiessling & Anderson (2003) *Human Embryonic Stem Cells: an introduction to the science and therapeutic potential.* ISBN: 076372341X.
[10] Donovan & Gearhart (2001) *Nature* 414:92-97.
[11] Pera (2001) *Curr Opin Genet Dev.* 11:595-599.
*[12] Stem cells and the future of regenerative medicine* (2002) ISBN: 0-309-07630-7.
[13] *The human embryonic stem cell debate: science, ethics and public policy* (2001) eds. Holland, Lebacqz & Zoloth. ISBN: 0262582082.
[14] McLaren (2001) *Nature* 414:129-131.
[15] Waite & Nindl (2003) *Biomed Sci Instrum* 39:567-572.
[16] International patent application PCT/ES02/00116.
[17] Kattera & Chen (2003) *Human Reproduction* 18:1319-1322.
[18] Malter & Cohen (1989) *Fertil. Steril.* 52:373-380.
[19] Tang *et al.* (1994) *Zygote* 1:79-85.
[20] Simon *et al.* (1999) *J Clin Endocrinol Metab* 84:2638-2646.
[21] Cheng *et al.* (2003) *Stem Cells* 21:131-142.
[22] Richards *et al.* (2002) *Nature Biotechnol* 20:933-936.
[23] Hovatta *et al.* (2003) *Hum Reprod* 18:1404-1409.
[24] Amit *et al.* (2003) *Biol Reprod* 68:2150-2156.
[25] WO03/040346.
[26] Kleinman *et al.* (1982) *Biochemistry* 21:6188-6193.
[27] Xu *et al.* (2001) *Nature Biotechnol* 19:971-974.
[28] WO01/51616.
[29] WO03/020920.
[30] Gibco *Knockout™ DMEM medium.* Invitrogen catalog no. 10829.
[31] Gibco *Knockout™ serum replacement.* Invitrogen catalog no. 10828.
[32] Reubinoff *et al.* (2000) *Nature Biotechnol* 18:399-404.
[33] *Differentiation of embryonic stem cells.* (2003) *ed.* Wassarman. ISBN: 0121822680.
[34] Carpenter *et al.* (2003) *Cloning Stem Cells* 5:79-88.
[35] Schuldiner *et al.* (2000) *PNAS USA* 97:11307-11312.
[36] Odorico *et al.* (2001) *Stem Cells* 19:193-204.
[37] Desbaillets *et al.* (2000) *Exp Physiol* 85:645-51.
[38] Keller (1995) *Curr Opin Cell Biol* 7:862-9.
[39] Mueller-Klieser (1997) *Am J Physiol* 273:C 1109-23.
[40] Magyar *et al.* (2001) *Ann N Y Acad Sci* 944:135-43.
[41] Zhang *et al.* (2001) *Nature Biotechnol* 19:1129-33.
[42] Itskovitz-Eldor *et al.* (20002) *Mol Med* 6:88-95.
[43] Schuldiner *et al.* (2001) *Brain Res* 913:201-205.
[44] Kehat *et al.* (2001) *J Clin Invest* 108:407-414.
[45] Eiges *et al.* (2001) *Curr Biol* 11:514-518.
[46] US patent application 2002/0019046.
[47] WO01/65928.
[48] Zamore (2001) *Nat Struct Biol* 8:746-750.
[49] Carthew (2001) *Curr Opin Cell Biol* 13:244-248.
[50] WO03/012082.
[51] Billy *et al.* (2001) *PNAS USA* 98:14428-14433.
[52] Yang *et al.* (2001) *Mol Cell Biol* 21:7807-7816.
[53] Ingram *et al.* (1997) *In Vitro Cell Dev Biol Anim* 33:459-466.
[54] US patent 6,458,589 (see also WO01/81549).
[55] Rambhatla *et al.* (2003) *Cell Transplant* 12:1-11.
[56] US patent application 2003/0068819.
[57] Studer (2001) *Nature Biotechnol* 19:1117-1118.
[58] Carpenter *et al.* (2001) *Exp Neurol* 172:383-397.
[59] Reubinoff *et al.* (2001) *Nature Biotechnol* 19:1134-1140.
[60] Goldstein *et al.* (2002) *Dev Dyn* 225:80-86.
[61] WO03/048344.
[62] US patent application 2003/0103949.
[63] Vacario & Schimmang (2003) *J Neurosci Meth* 123:55-60.
[64] Kaufman *et al.* (2001) *PNAS USA* 98:10716-10721.
[65] WO01/34776.
[66] US patent 6,280,718.
[67] WO03/050251 (see also US patent application 2003/0153082).
[68] Chadwick *et al.* (2003) *Blood* DOI 10.1182/blood-2003-03-0832
[69] Levenberg *et al.* (2002) *PNAS USA* 99:4391-4396.
[70] WO03/040319.
[71] Unknown author(s) (2003) *Ontogenez* 34:204-210.
[72] US patent application 2003/0022367.
[73] Xu *et al.* (2002) *Circ Res* 91:501-508.
[74] Takahashi *et al.* (2003) *Circulation* 107:1912-1916.
[75] WO03/010303.
[76] Mummery *et al.* (2003) *Circulation* 107:2733-2740 (see also pages 2638-2639).
[77] Assady *et al.* (2001) *Diabetes* 50:1691-1697.
[78] Moritoh *et al.* (2003) *Diabetes* 52:1163-1168.
[79] WO03/050249 (see also US patent application 2003/0138948).
[80] Xu *et al.* (2002) *Nature Biotechnol* 20:1261-1264.
[81] WO01/88100.
[82] WO02/097068.
[83] WO01/98463 (see also US patent application 2002/022267).
[84] Sottile *et al.* (2003) *Cloning Stem Cells* 5:149-155.
[85] WO03/004605 (see also US patent applications 2003/0021771 & 2003/0036194).
[86] WO03/050250 (see also US patent application 2003/0109038).
[87] WO03/060062 (see also US patent application 2003/0148510).
[88] US patent 5,888,720.
[89] WO02/14469.
[90] Freed (2002) *PNAS USA* 99:1755-1757.
[91] Björklund *et al.* (2002) *PNAS USA* 99:2344-2349.
[92] US patent 6,534,052.
[93] Kehat & Gepstein (2003) *Heart Fail Rev* 8:229-236.
[94] Nir *et al.* (2003) *Cardiovasc Res* 58:313-323.
[95] Gennaro (2000) *Remington: The Science and Practice of Pharmacy.* 20th ed., ISBN: 0683306472.
[96] *Cell Therapy : Stem Cell Transplantation, Gene Therapy, and Cellular Immunotherapy* (eds. Morstyn & Sheridan). ISBN: 0521473152.
[97] Siebers *et al.* (1997) *Ann N Y Acad Sci* 831:304-12.
[98] US patent 5,837,234.
[99] Lacy *et al.* (1991) *Science* 254:1782-84.
[100] Sakai *et al.* (2001) *Ann N Y Acad Sci* 944:277-83
[101] *Synthetic biodegradable polymer scaffolds* (Atala & Mooney). ISBN: 0817639195.
[102] Vats *et al.* (2003) *Clin Otolaryngol* 28:165-172.
[103] Tuli *et al.* (2003) *Arthritis Res Ther* 5:235-238.
[104] Shin *et al.* (2003) *Biomaterials* 24:4353-4364.
[105] Pavesio *et al.* (2003) *Novartis Found Symp* 249:203-217 & 229-241.
[106] Vunjak-Novakovic (2003) *Novartis Found Symp* 249:34-46, 46-51, 170-174 & 239-241.
[107] Takezawa (2003) *Biomaterials* 24:2267-2275.
[108] Griffith (2002) *Ann N Y Acad Sci* 961:83-95.
[109] Chaikof *et al.* (2002) *Ann N Y Acad Sci* 961:96-105.
[110] Hodde *et al.* (2002) *Tissue Eng* 8:295-308.
[111] Drukker *et al.* (2002) *PNAS USA* 99:9864-9869.
[112] Rohwedel *et al.* (2001) *Toxicol In Vitro* 15:741-753.
[113] Wobus & Hescheler (1992) *ALTEX* 9:29-42.
[114] Bremer *et al.* (2001) *Toxicol In Vitro* 15:215-223.
[115] Scholz *et al.* (1999) *Cells Tissues Organs* 165:203-211.
[116] Vogel (1993) *Reprod Toxicol* 7 Suppl 1:69-73.
[117] *In Vitro Methods in Pharmaceutical Research* (eds. Castell, Gomez-Lechon & Gomes-Lochen). ISBN: 012163390X (1st edition, 1997).
[118] Lavon & Benvenisty (2003) *Trends Cardiovasc Med* 13:47-52.
[119] Gropp *et al.* (2003) *Mol Ther* 7:281-287.
[120] Pfeifer *et al.* (2002) *PNAS USA* 99:2140-2145.
[121] Ma *et al.* (2003) *Stem Cells* 21:111-117.
[122] Springer & Niculescu-Duvaz (2000) *J Clin Invest* 105:1161-1167.
[123] Schuldiner *et al.* (2003) *Stem Cells* 21:257-265.
[124] WO01/29206.
[125] Ledermann (2000) *Exp Physiol* 85:603-613.
[126] Denning & Priddle (2003) *Reproduction* 126:1-11.
[127] Zwaka & Thomson (2003) *Nature Biotechnol* 21:319-321.
[128] WO02/18549.
[129] Gil-Salom *et al.* (1996) *Hum Reprod* 6:1309-1313.
[130] Wiker *et al.* (1990) *J In Vitro Fertil Embryo Transfer* 7:33-37.
[131] Palermo *et al.* (1994) *Hum Reprod* 9:1220-1225.
[132] Munné *et al.* (1993) *Hum Reprod* 8:2185-2191.
[133] Willadsen (1986) *Nature* 320:63-65.
[134] Smith & Wilmut (1989) *Biol Reprod* 40:1027-1035.
[135] Collas & Robl (1990) *Biol Reprod* 43:877-884.
[136] Rawlins *et al.* (1988) *Fertil Steril 50:266-272.*
[137] Gordon *et al.* (1989) *Fertil Steril* 52:367-372.
[138] Plachot & Crozet (1992) *Hum Reprod* 7:89-94.
[139] Plachot & Mandelbaum (1990) *British Med Bull* 46:675-694.
[140] Sathananthan *et al.* (1999) *Hum Reprod Update* 5:553-560.
[141] Rosenbusch *et al.* (1997) *Hum Reprod* 10:2257-2262.
[142] Rosenbusch *et al.* (1998) *Mol Hum Reprod* 11:1065-1070.
[143] Lawler *et al.* (1991) *Am J Obstet Gynecol* 164:1270-1277.
[144] Edwards *et al.* (1990) *Lancet* 335: 1030-1031.
[145] Niemierko & Opas (1978) *Manipulation of ploidy in the mouse.* In: Daniel JC (ed) *Methods in Mammalian Reproduction.* New York: Academic Press, 1978; 50-65.
[146] Surani *et al.* (1986) *Cell* 45:127-136.
[147] Comey *et al.* (1994) *J Forensic Sci* 39:1254-1269.
[148] Akane *et al.* (1994) *J Forensic Sci* 39:362-372.

## Claims

1. A method for preparing embryonic stem cells, comprising the steps of: (a) providing a tripronucleated zygote; (b) removing one of the pronuclei to provide a diploid heteroparental zygote; (c) culturing the diploid zygote to produce a blastocyst; (d) obtaining one or more cells from the inner cell mass of the blastocyst; and (e) culturing the cells obtained from the inner cell mass to obtain embryonic stem cells.

2. The method of claim 1, wherein the zygote is a human zygote.

3. The method of any preceding claim, wherein the zygote is treated with a cytoskeletal relaxant prior to step (b).

4. The method of any preceding claim, wherein step (e) involves *in vitro* culture of ICM cells on a feeder layer of cells.

5. The method of any one of claims 1 to 3, wherein step (e) involves *in vitro* culture of ICM cells without a feeder layer of cells.

6. A method for preparing a desired cell, comprising steps (a) to (e) as described in any one of claims 1 to 5, and the further step of: (f) differentiating the ES cell into the desired cell.

7. The method of claim 6, wherein step (f) involves the production of an embryoid body (EB).

8. The method of claim 6 or claim 7, wherein the desired cell is from the endoderm, mesoderm or ectoderm layer.

9. The method of any one of claims 6 to 8, wherein the desired cell is selected from the group consisting of: neural cells; cardiac cells; intestinal cells; stomach cells; oesophageal cells; retinal cells; corneal cells; cartilage; bone cells; hematopoietic cells; muscle cells; lung cells; kidney cells; pancreatic cells; islet cells; glucose-sensitive insulin-secreting cells; brain cells; epithelial cells; endothelial cells; epidermis; cardiomycocytes; hematopoietic progenitors; yolk sac; skeletal myocytes; smooth muscle cells; fibroblasts; adipocytes; chondrocytes; melanocytes; neural precursors; neurons, including dopaminergic neurons, serotonergic neurons, cholinergic neurons, GABAergic neurons, sensory neurons, and motor neurons; glia; astrocytes; oligodendrocytes; hepatocytes; osteocytes; osteoblasts; monocytes; dendritic cells; lymphocytes; erythrocytes; leukocytes; NK cells; macrophages; erythroid cells; granulocytes; megakaryocytes; pneumocytes; comeocytes; trophoblast; keratinocytes; testis; ovary; cell lines; conjunctiva; limbal stem cells; mucosal cells; and lachrymal cells.

10. The method of any one of claims 6 to 8, wherein the desired cell expresses one or more markers selected from the group consisting of: core binding factor α1, collagen 1, osteocalcin, opteopontin, bone sialoprotein, parathyroid hormone receptor, bone/liver/kidney alkaline phosphatase, microtubule-associated protein 2, βIII tubulin, neurofilament glial fibrillary acidic protein, galactocerebroside, myelin basic protein, nestin, cardiac specific troponin, sMHC, tropomyosin, α-actinin, desmin, creatine kinase MB, myoglobin, GATA 4, MEF 2, Nkx2.5, atrial natriuretic factor, cardiac troponin T, albumin, α1-antitrypsin, CD18, cytokeratin 18, cytokeratin 8, cytokeratin 19, vimentin, nestin, polysialylated NCAM, A2B5, PECAM1, VE cad, GATA 2, CD34, pdx1, Ngn3, insulin, IAPP, Nkx6.1, transcription factor AP 2, msh homeobox homolog 2, suppressor of cytokine signalling 3, GATA binding protein 2, GATA binding protein 3, hairy/enhancer-of-split related with YRPW motif 1, keratin, aggrecan, type II collagen, osteocalcin, and type X collagen.

11. The method of any one of claims 6 to 10, wherein step (f) involves culturing the embryonic stem cell in the presence of one or more of the substances selected from the group consisting of: tissue necrosis factor α (TNF-α); TNF-β; interferon α (IFN α); IFN β; IFN *γ,* one or more interleukins (IL), including IL 1, IL 2, IL 3, IL 4, IL 5, IL 6, IL 7, IL 8, IL 9, IL 10, IL 11, IL 12, IL 13, IL 14, IL 15, IL 16, IL 17 & IL 18; fibroblast growth factors (FGF), including FGF 1 (or acidic FGF), FGF 2 (or basic FGF), FGF 3, FGF 4, FGF 5, FGF 6, FGF 7 (or keratinocyte growth factor), FGF 8, FGF 9, FGF 10, FGF 11, FGF 12, FGF 13, FGF 14, FGF 15, FGF 16, FGF 17, FGF 18, FGF 19, FGF 20, FGF 21, FGF 22 and FGF 23; transforming growth factor α (TGF α); transforming growth factor β (TGF β) 1 or 2, or a 1.2 heterodimer; bone morphogenic proteins (BMPs), including BMP 2 and BMP 4; hepatocyte growth factor (HGF); epidermal growth factor (EGF); β nerve growth factor (NGF); neurotrophins, such as NT 3; brain derived neurotrophic factor (BDNF); activin A; vascular endothelial cell growth factor (VEGF); granulocyte colony stimulating factor (GCSF); granulocyte macrophage colony stimulating factor (GMCSF); platelet derived growth factor (PDGF); insulin; insulin-like growth factor I (IGF 1); IGF 2; erythropoietin (Epo); stem cell factor (SCF); glucagons; glucagons-like peptide 1 (GLP 1); Flt 3 ligand; hedgehog proteins, such as sonic hedgehog; Ca²⁺; Mg²⁺; pyruvate; butyrate; retinoic acid; fibroblast growth factor 2 (FGF 2); dimethylsulfoxide (DMSO); hexamethylene bisacetamide (HMBA); glucocorticoids, such as dexamethasone; extracellular matrix-promoting agents, such as ascorbic acid; cell extracts; hydrocortisone; catecholamines; epinephrine; mineralisation promoters; β-glycerophosphate; triiodothyronine; a demethylation reagent; 5-aza-2'-deoxycytidine; serum albumins; antibiotics; antifungals; and amphotericin B.

12. A method for preparing a desired cell, comprising steps (a) to (e) as described in any one of claims 1 to 5, and the further step of: (f) culturing a starting cell with the ES cell obtained in step (e) to transdifferentiate the starting cell into the desired cell.

13. An embryonic stem (ES) cell derived from a tripronucleated zygote.

14. The ES cell of claim 13, which is a human ES cell obtained using the method of claim 1.

15. The ES cell of claim 14, wherein the ES cell has a stable karyotype, has 23 pairs of chromosomes (including XX or XY), has a prolonged ability to divide symmetrically without differentiation, has an ability to give rise to differentiated cell types from all three primary germ layers; prolonged telomerase activity, displays of markers of non differentiation and is non-tumorigenic.

16. The ES cell of any one of claims 13 to 15, wherein the ES cell is pluripotent and cannot give rise to an embryo.

17. The use of an ES cell according to any one of claims 13 to 16 for transdifferentiation of a human cell *in vitro* or *in vivo.*

18. A differentiated cell obtained by the method of any one of claims 6 to 12.

19. The cell of any one of claims 13 to 18, for use in medicine.

20. The use of a cell of any one of claims 13 to 18 in the manufacture of a medicament.

21. A method of therapy, comprising the step of delivering the cell of any one of claims 13 to 18 to a patient.

22. The method of claim 21, wherein the method is for treating insulin-dependent diabetes, Parkinson's disease, Huntington's disease, spinal cord injury, amyotrophic lateral sclerosis, Alzheimer's disease, myocardial infarction, ischemic cardiac tissue or heart failure; side effects of radiation; corneal scarring; liver cirrhosis or failure; ischemic brain damage; spinal cord damage; cartilage damage; bone damage; osteoarthritis; myelination disorders, such as Pelizaeus-Merzbacher disease, multiple sclerosis, adenoleukodystrophies, neuritis and neuropathies.

23. A pharmaceutical composition comprising a cell of any one of claims 13 to 18.

24. A process for preparing a secreted factor of interest, comprising steps (a) to (f) as described in claim 6, and further comprising the steps of: (g) culturing the cells obtained in step (f) in a culture medium under conditions such that they produce the factor of interest and secrete it into the culture medium; and (h) purifying the factor from the culture medium.

25. A method for preparing nucleic acid from a cell, comprising steps (a) to (f) as described in claim 6, and further comprising the step either of: (g1) preparing genomic DNA from a cell obtained in step (f), or (g2) preparing mRNA from a cell obtained in step (f) and, optionally, preparing cDNA from said mRNA.

26. A process for preparing a panel of cells, tissues or organs of different cell types, comprising steps (a) to (f) as defined in claim 6, wherein at least step (f) is performed at least twice, thereby providing the panel.

27. A process for preparing a panel of cells of the same cell type, comprising at least two separate runs of steps (a) to (f) as defined in claim 6, wherein each run uses a different tripronucleated zygote in step (a).

28. A panel of cells, obtained by the process of claim 26 or claim 27.

29. The panel of claim 27 or claim 28, comprising one or more of the following cell types: hepatocytes; comeocytes; corneal tissue; epidermis; keratinocytes; fibroblasts; kidney cells; lung tissue; pneumocytes; lymphocytes; neurons; testis; and ovary.

30. The panel of any one of claims 27 to 29, for use in assessing toxic effects, metabolism, allergic reactions, side effects, biodistribution, inflammatory reactions, contact reactions, or dermatological effects or a metarial.

31. A process for testing the effect(s) of a test material on a cell of interest, comprising preparing a panel of cells of the same or different cell type according to any one of claims 26 to 30, and further comprising the steps of: (g) contacting the cells obtained from step (f) with the test material; and (h) detecting any effects on the cells arising from step (g).

32. An *in vitro* screening assay, comprising steps (a) to (f) as defined in claim 6, further comprising steps: (g) contacting the cell obtained in step (f) with a test compound; and (h) detecting in a cell the presence or absence of a change arising from step (g).

33. An *in vitro* screening assay, comprising steps (a) to (e) as defined in claim 6, and further comprising steps: (f) contacting the ES cell obtained in step (e) with a test compound and/or exposing the ES cell to altered environmental conditions; and (g) detecting in a cell the presence or absence of differentiation.

34. A method for preparing a genetically-modified ES cell, comprising steps (a) to (e) as defined in any one of claims 1 to 5, and further comprising the step: (f) genetically modifying the ES cell.
